# EUROPEAN PATENT APPLICATION

(11) **EP 4 806 316 A1**
(43) Date of publication of application: **16.09.2026**
(21) Application number: 25205901.9
(22) Date of filing: 30.09.2025
(51) Int. Cl.: A47L 7/00, A47L 9/00, A47L 9/28, A47L 11/40, A61L 2/10

(54) **DOCKING STATION AND CLEANING SYSTEM**

(30) Priority: 11.03.2025 CN 202520421156 U
(71) Applicant: Beijing Xiaomi Mobile Software Co., Ltd., Beijing 100085 (CN)
(72) Inventor: GE, Ning, Beijing, 100085 (CN)
(74) Representative: Dehns Germany Partnerschaft mbB

(57) **Abstract**

A docking station (1) and a cleaning system. The docking station (1) includes: a housing (11) provided with a dust collection chamber (111); a dust collection duct (12) connected to the dust collection chamber (111); a fan (13) connected to both the dust collection chamber (111) and the dust collection duct (12), the fan (13) being used to circulate air in the dust collection chamber (111) and the dust collection duct (12); and an antibacterial module (14) at least partially located within the dust collection duct (12).

## Description

### TECHNICAL FIELD

This application relates to the field of the cleaning device technology, specifically to, a docking station and a cleaning system.

### BACKGROUND

Currently, a cleaning part of a cleaning device such as a robotic vacuum cleaner on the market generally has sweeping and mopping functions. During the working process thereof, dust, hair, and wastewater are sucked into a dust collection box on the cleaning part of the robotic vacuum cleaner. Additionally, to reduce a use-cost of a user, a dust collection function becomes increasingly common to a docking station of the robotic vacuum cleaner. The docking station may store the debris collected by the cleaning part of the robotic vacuum cleaner for a long time. Both the docking station and the cleaning part of the robotic vacuum cleaner may generate heat during the working process thereof, which causes a dust collection path to remain in a humid and warm environment for a long time. Such environment is highly conducive to bacterial growth and unpleasant odor production, which may pollute a home environment and significantly impair a user experience.

It should be noted that, information disclosed in the above background portion is provided only for better understanding of the background of the present invention, and thus it may contain information that does not form the prior art known by those ordinary skilled in the art.

### SUMMARY

In view of the above, the present invention provides a docking station and cleaning system for improving the antibacterial effect of the dust collection path.

Specifically, the following solutions are provided.

In a first aspect, the present invention provides a docking station, including:
a housing provided with a dust collection chamber;
a dust collection duct connected to the dust collection chamber;
a fan connected to both the dust collection chamber and the dust collection duct, the fan being used to circulate air in the dust collection chamber and the dust collection duct; and
an antibacterial module at least partially located within the dust collection duct.

In an optional embodiment, the antibacterial module includes one or more of a negative ion generator, a disinfectant sprayer, a disinfectant sustained-release device, and an ultraviolet radiator.

In an optional embodiment, the dust collection duct includes a dust suction duct and a blowing duct, the dust suction duct and the blowing duct are respectively connected to the dust collection chamber, an air inlet end of the fan is connected to the dust collection chamber, and an air outlet end of the fan is connected to the blowing duct.

In an optional embodiment, the antibacterial module includes a main body, an output line, and a release tip, the main body is mounted on the housing, two ends of the output line are respectively connected to the main body and the release tip, and the release tip extends into the dust collection duct.

In an optional embodiment, the main body is located at a side of the blowing duct, and the release tip extends into the blowing duct.

In an optional embodiment, the antibacterial module further includes a mounting bracket, the mounting bracket is fixed to an outer wall of the blowing duct, and an end of the output line close to the release tip is inserted into the mounting bracket, so that the release tip passes through the mounting bracket and extends into the blowing duct.

In an optional embodiment, the antibacterial module is a negative ion generator, the output line is a high-voltage output line, the main body includes a current conversion module connected to a power supply and configured to convert an input direct current or alternating current into negative high voltage direct current, the output line is configured to transmit the negative high voltage direct current to the release tip so that the release tip generates an electric corona.

In an optional embodiment, the antibacterial module has a business end configured to generate an antibacterial substance or perform antibacterial operation, and the business end is located within the dust suction duct or the blowing duct.

In an optional embodiment, the docking station includes a plurality of the antibacterial modules, the business ends of a part of the antibacterial modules are extended into the blowing duct, and the business ends of the other part of the antibacterial modules are extended into the dust suction duct.

In an optional embodiment, the docking station has a dust collection mode and an antibacterial mode, the fan is configured to switch between the dust collection mode and the antibacterial mode, the antibacterial module is configured to be activated at least in the antibacterial mode, and a power of the fan in the dust collection mode is greater than a power of the fan in the antibacterial mode.

In an optional embodiment, the docking station further includes a cover, and the cover covers an opening of the dust collection chamber, and is rotatably or removably connected to the housing to open or close the dust collection chamber.

In a second aspect, the present invention provides a cleaning system, including a cleaning device and the docking station according to any embodiment in the first aspect, the cleaning device includes a dust collection box, and the dust collection box is adapted to be connected to the dust collection duct of the docking station.

In an optional embodiment, the dust collection duct includes a dust suction duct and a blowing duct, the dust suction duct and the blowing duct are respectively connected to the dust collection chamber, an air inlet end of the fan is connected to the dust collection chamber, and an air outlet end of the fan is connected to the blowing duct; and

the dust collection box is provided with an air inlet and an air outlet, the air inlet is used to connect with the blowing duct, and the air outlet is used to connect with the dust suction duct.

In an optional embodiment, the housing further includes an accommodation section at the bottom of the dust collection chamber, and the accommodation section has a shape matching a shape of the cleaning device, so that the cleaning device can rest against the accommodation section.

The beneficial effects of the technical solution provided by the embodiments of the present invention include at least the following: the antibacterial module is at least partially provided within the dust collection duct, which prevents the antibacterial module from directly contacting with or being covered by debris in the dust collection chamber and thus can improve the antibacterial effect; and under the driving force of the fan, air circulates and flows within the dust collection chamber and the dust collection duct, enabling the antibacterial module to continuously perform an antibacterial treatment on the air within the dust collection chamber and the dust collection duct, which ensures a more comprehensive and thorough antibacterial effect, effectively guarantees a healthy environment within the docking station, and thus enhances the user experience.

### BRIEF DESCRIPTION OF THE DRAWINGS

To more clearly illustrate the technical solutions in the embodiments of the present invention, the following provides a brief introduction to the figures used in the description of the embodiments. Obviously, the figures described below are merely some embodiments of the present invention, a person skilled in the art may obtain other figures based on these figures without creative labor.
FIG. 1 is a schematic diagram of a structure of a docking station provided by an embodiment of the present invention;
FIG. 2 is a schematic diagram of a structure of a docking station when a cover thereof is removed provided by an embodiment of the present invention;
FIG. 3 is a schematic diagram of an airflow path between a docking station and a cleaning device provided by an embodiment of the present invention;
FIG. 4 is another schematic diagram of an airflow path between a docking station and a cleaning device provided by an embodiment of the present invention; and
FIG. 5 is a schematic diagram of a structure of an antibacterial module provided by an embodiment of the present invention.

Brief description of reference numerals:
1-Docking station;
11-Housing; 111-Dust collection chamber; 112-Accommodation section; 12-Dust collection duct; 121-Dust suction duct; 122-Blowing duct; 13-Fan; 14-Antibacterial module; 141-Main body; 142-Output line; 143-Release tip; 144-Mounting bracket; 15-Cover;
2-Cleaning device; 21-Dust collection box; 211-Air inlet; 212-Air outlet; 22-Dust inlet cover; 23-Filter mesh.

### DETAILED DESCRIPTION

The technical solution in the embodiments of the present invention will be clearly and completely described below with reference to the drawings in the embodiments of the present invention. Obviously, the described embodiments are merely some embodiments but not all embodiments of the present invention.

The orientation terms used in the present disclosure such as "on", "under", and "at a side of ..." generally refer to the relative positions shown in the drawings. These orientation terms are used solely for clearly describing a relationship between structures, and not for describing absolute orientations. When a product is placed in different arrangements, the orientation thereof may change, for example, "on" and "under" may be interchanged.

Unless otherwise defined, all technical terms used in the present disclosure have the same meanings as those commonly understood by a person skilled in the art. The following provides an explanation of some technical terms in the present disclosure.

To make the technical solutions and advantages of the present invention clearer, the following will provide a further detailed description of the embodiments of the present invention with reference to the accompanying drawings.

In the related art, some robotic vacuum cleaners are provided with an antibacterial device, which is typically arranged inside a dust collection container of the docking station. The antibacterial device continuously releases antibacterial substances to perform antibacterial treatments on dirt, dust, food residues within the container. A dust collection operation may easily contaminate the entire dust collection path including a dust collection duct, a dust bag in the cleaning part of the robotic vacuum cleaner, and a dust collection container in the docking station. In current designs, the antibacterial treatment is mainly directed to the dust collection container in the docking station. However, the antibacterial device may be contaminated or covered by debris in the dust collection container, resulting in a poor antibacterial effect of the entire dust collection path.

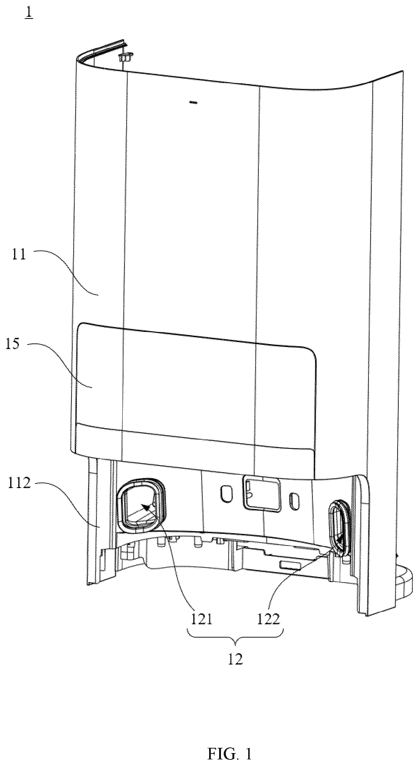

As shown in FIGS. 1 to 3, an embodiment of the present invention provides a docking station 1 including a housing 11, a dust collection duct 12, a fan 13, and an antibacterial module 14.

The housing 11 is provided with a dust collection chamber 111, the dust collection duct 12 is connected to the dust collection chamber 111, and the fan 13 is connected to both the dust collection chamber 111 and the dust collection duct 12. The fan 13 is used to circulate air in the dust collection chamber 111 and the dust collection duct 12. The antibacterial module 14 is at least partially located within the dust collection duct 12.

Specifically, the docking station 1 is adapted to use in conjunction with a cleaning device 2. The cleaning device 2 may include but is not limited to a sweeping robot, a mopping robot, or a floor-washing machine. The docking station 1 is fixed in a position and is used to charge the cleaning device 2, refill the cleaning device 2 with water, clean/empty a dust collection box 21, wash a mop or the like. The cleaning device 2 may move within an environment to clean a floor, and may also be charged, refilled with water and maintained at the docking station 1.

The dust collection duct 12 is adapted to connect with the dust collection box 21 of the cleaning device 2. Under the driving of the fan 13, air enters into the dust collection box 21 of the cleaning device 2 and carries away debris inside the dust collection box 21, and the debris is collected into the dust collection chamber 111 of the docking station 1 through the dust collection duct 12, thereby emptying the dust collection box 21 of the cleaning device 2.

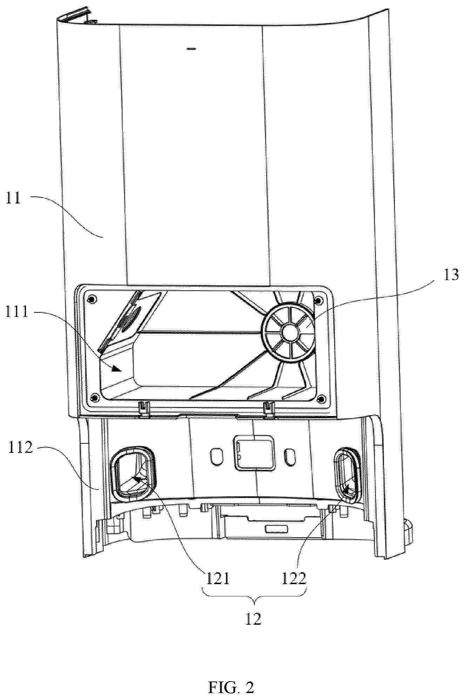

Optionally, as shown in FIG. 1 or 2, the housing 11 further includes an accommodation section 112 at the bottom of the dust collection chamber 111. The accommodation section 112 has an arcuate shape that matches a cylindrical shape of the cleaning device 2, allowing the cleaning device 2 to rest against the front of the accommodation section 112. The dust collection duct 12 passes through the accommodating section 112 to connect with the dust collection box 21 of the cleaning device 2.

In the related art, most docking stations on the market are provided with an antibacterial device in a dust collection chamber to prevent bacterial growth or unpleasant odor production on the debris in the dust collection chamber. However, such design occupies a space in the dust collection chamber, reducing the volume thereof. Additionally, the antibacterial device is prone to contact with or being covered by debris in the dust collection chamber, resulting in a poor antibacterial effect, and thus may not provide comprehensive antibacterial treatment for the entire dust collection path.

In the embodiment of the present invention, the antibacterial module 14 is at least partially provided within the dust collection duct 12, which can prevent the antibacterial module 14 from directly contacting with or being covered by debris in the dust collection chamber 111 and thus can improve the antibacterial effect; and under the driving force of the fan 13, air circulates and flows within the dust collection chamber 111 and the dust collection duct 12, enabling the antibacterial module 14 to continuously perform an antibacterial treatment on the air within the dust collection chamber 111 and the dust collection duct 12, which ensures a more comprehensive and thorough antibacterial effect, effectively guarantees a healthy environment within the docking station 1, and thus enhances the user experience.

It is to be understood that the business end of the antibacterial module 14 is located within the dust collection duct 12. The business end refers to the portion of the antibacterial module 14 for generating antibacterial substances or performing antibacterial operations, with which the antibacterial module 14 is ensured to function normally to perform the antibacterial treatment on the dust collection chamber 111 and dust collection duct 12.

The antibacterial module 14 may be an electrically powered device or a non-electrically powered device.

In a specific embodiment, the antibacterial module 14 includes one or more of a negative ion generator, a disinfectant sprayer, a disinfectant sustained-release device, and an ultraviolet radiator.

The negative ion generator ionizes oxygen molecules in the air by applying a high voltage, thereby generating a large number of negative oxygen ions. These negative oxygen ions have high activity and can bind to proteins on the surface of bacteria, this can alter potential of the bacteria or damage the cell wall of the bacteria, thereby disrupting the structure and function of the bacteria. This action renders the bacteria incapable of survival, achieving an antibacterial effect. Additionally, the negative ions may also accelerate the settling of dust in the air. The negative ions carry negative charges and thus adsorb and remove harmful substances such as particles and bacteria from the air. These particles and bacteria typically carry positive charges or are electrically neutral, and thus are attracted to the negative ions, thereby reducing the concentration and quantity of the particles and bacteria in the air.

Disinfectant may specifically be chlorine dioxide, ozone, sodium chloride, or ammonium chloride solution spray, etc.

The disinfectant sprayer may atomize liquid disinfectant into tiny particles, allowing the disinfectant to be distributed more evenly throughout the space.

The disinfectant sustained-release device may continuously release disinfectant at a slow rate over a certain period of time, allowing the disinfectant to cover a specific space so as to inhibit bacterial growth.

The ultraviolet radiator may emit ultraviolet ray (particularly in the UVC band), which can be absorbed by DNA and RNA of a microorganism, causing a change in the molecular structure of the microorganism, thereby preventing replication and reproduction of the microorganism. Additionally, when the ultraviolet ray acts on air, oxygen is ionized to produce ozone with strong oxidation, which can effectively kill the microorganism.

Optionally, there may be one or more antimicrobial modules 14. When there are a plurality of antimicrobial modules 14, the plurality of antimicrobial modules 14 may include one of the negative ion generator, the disinfectant sprayer, the disinfectant sustained-release device, and the ultraviolet radiator, or may include a combination of two or more thereof.

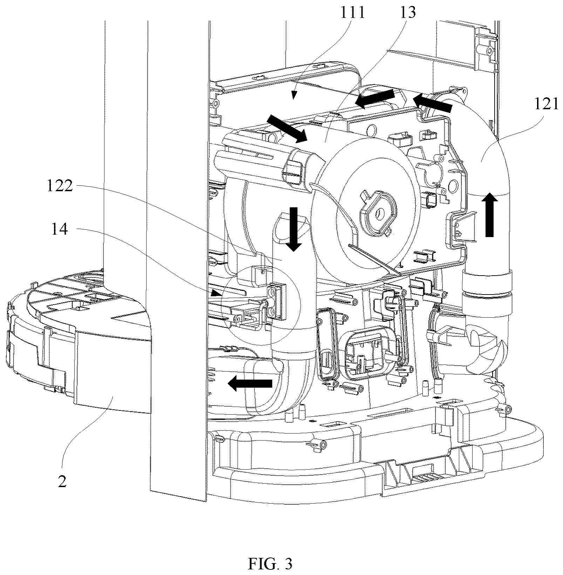

In an embodiment, as shown in FIG. 2 or 3, the dust collection duct 12 includes a dust suction duct 121 and a blowing duct 122. The dust suction duct 121 and the blowing duct 122 are respectively connected to the dust collection chamber 111. An air inlet end of the fan 13 is connected to the dust collection chamber 111, and an air outlet end of the fan 13 is connected to the blowing duct 122.

As shown in FIGS. 2 and 3, the dust suction duct 121, the blowing duct 122, and the fan 13 are all located on a back side of the housing 11. The dust suction duct 121 and the blowing duct 122 are spaced apart along a width direction of the housing 11, and the fan 13 and an end of the dust suction duct 121 connected to the dust collection chamber 111 are spaced apart along the width direction of the housing 11 to prevent debris entering into the dust collection chamber 111 from being sucked into the blowing duct 122. It is to be noted that the width direction of the housing 11 refers to a direction from a left end to a right end of the dust collection chamber 111 in FIG. 2.

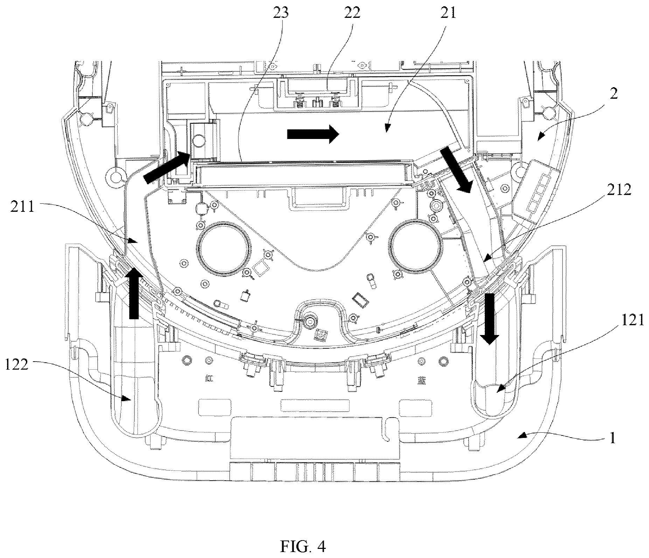

Referring to FIGS. 3 and 4, when the docking station 1 is collecting dust, the fan 13 is activated, and a negative pressure is created inside the dust collection chamber 111 to output an air flow to the blowing duct 122. The air flow enters into the dust collection box 21 from an air inlet 211 of the cleaning device 2, and carries debris away from the dust collection box 21. The debris is collected into the dust collection chamber 111 of the docking station 1 through an air outlet 212 of the cleaning device 2 and the dust suction duct 121. The air flow circulates along and through the blowing duct 122, the dust collection box 21 of the cleaning device 2, the dust suction duct 121, and the dust collection chamber 111.

The business end of the antibacterial module 14 may be extended into the blowing duct 122 or into the dust suction duct 121. Optionally, when there are a plurality of antibacterial modules 14, the business ends of some of the antibacterial modules 14 are extended into the blowing duct 122, and the business ends of the other antibacterial modules 14 are extended into the dust suction duct 121, which may further enhance the antibacterial effect.

For example, when the antibacterial module 14 is at least partially located within the blowing duct 122, under the action of the antibacterial module 14, the air flow within the blowing duct 122 may carry antibacterial substances such as negative ions to flow through the blowing duct 122, the dust collection box 21 of the cleaning device 2, the dust suction duct 121, and the dust collection chamber 111, enabling the air flow carrying the antibacterial substances to be circulated, thereby achieving comprehensive and thorough antibacterial treatment.

A dust collection path is formed between the cleaning device 2 and the docking station 1 by providing the dust suction duct 121 and the blowing duct 122, so that the air flow carrying antibacterial substances may circulate between the cleaning device 2 and the docking station 1, thereby achieving comprehensive and thorough antibacterial treatment.

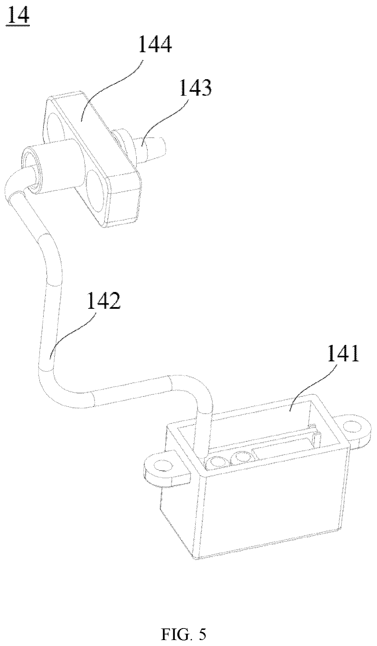

In a specific embodiment, as shown in FIG. 5, the antibacterial module 14 is a negative ion generator. The antibacterial module 14 includes a main body 141, an output line 142, and a release tip 143. The main body 141 is mounted on the housing 11, and two ends of the output line 142 are respectively connected to the main body 141 and the release tip 143. The release tip 143 extends into the dust collection duct 12.

As shown in FIG. 5, one end of the output line 142 is connected to the main body 141, and the other end thereof is connected to the release tip 143. Specifically, the output line 142 is a high-voltage output line. The main body 141 of the antibacterial module 14 is fixed on the back side of the housing 11. The main body 141 includes a current conversion module, which is connected to a power supply. The current conversion module converts the input direct current or alternating current into negative high voltage direct current. The output line 142 transmits the negative high voltage direct current to the release tip 143 made of metal or carbon. The high voltage direct current from the release tip 143 generates a high electric corona, thereby rapidly releasing a large number of electrons. However, the electrons may not persist in the air for a long time and are immediately captured by oxygen molecules in the air, thereby generating air negative ions.

The release tip 143 extends into the dust collection duct 12 to generate the negative ions, which render bacteria in the dust collection duct 12 incapable of survival, and at the same time, adsorb particles and bacteria, thereby achieving an antibacterial effect and accelerating dust settling.

In a specific embodiment, the main body 141 is located at a side of the blowing duct 122, and the release tip 143 extends into the blowing duct 122.

As shown in FIG. 3, the antibacterial module 14 is located near the blowing duct 122 to facilitate insertion of the release tip 143 into the blowing duct 122.

In this embodiment, the air flow within the blowing duct 122 may carry negative ions, and flow through the blowing duct 122, the dust collection box 21 of the cleaning device 2, the dust suction duct 121, and the dust collection chamber 111, enabling the air flow carrying antibacterial substances to be circulated, thereby achieving comprehensive and thorough antibacterial treatment.

Furthermore, as shown in FIG. 5, the antibacterial module 14 also includes a mounting bracket 144, which is fixed to an outer wall of the blowing duct 122. An end of the output line 142 close to the release tip 143 is inserted into the mounting bracket 144, so that the release tip 143 passes through the mounting bracket 144 and extends into the blowing duct 122.

The mounting bracket 144 is fixed to the outer wall of the blowing duct 122 via adhesive bonding, snap connection, or threaded connection. The output line 142 may be integrally connected to the mounting bracket 144, or may be separated from but fixed to the mounting bracket 144.

By providing the mounting bracket 144, the release tip 143 is prevented from shaking within the blowing duct 122, thereby enhancing the stability thereof. Additionally, the mounting bracket 144 provides some protection for the output line 142 to prevent the output line 142 from being damaged or broken due to excessive bending.

In an embodiment, the docking station 1 has a dust collection mode and an antibacterial mode, the fan 13 is configured to switch between the dust collection mode and the antibacterial mode, the antibacterial module 14 is configured to be activated at least in the antibacterial mode, and a power of the fan 13 in the dust collection mode is greater than a power of the fan 13 in the antibacterial mode.

Specifically, in the dust collection mode, the fan 13 is activated, the antibacterial module 14 is deactivated, an operating power of the fan 13 is a first power, and a rotational speed of the fan 13 is a first rotational speed; and in the antibacterial mode, both the fan 13 and the antibacterial module 14 are activated, the operating power of the fan 13 is a second power, and the rotational speed of the fan 13 is a second rotational speed. The first power is greater than the second power, and the first rotational speed is greater than the second rotational speed.

In an optional embodiment, the antibacterial module 14 may be activated in the dust collection mode to perform both the antibacterial operation and the dust collection, and can be also activated in the antibacterial mode. In another optional embodiment, the antibacterial module 14 is only activated in the antibacterial mode.

In the dust collection mode, the fan 13 operates at higher power and rotational speed to quickly collect debris from the dust collection box 21 of the cleaning device 2; and in the antibacterial mode, the fan 13 operates at lower power and rotational speed, which facilitates a smooth circulation of the air flow carrying antibacterial substances within the dust collection path, thereby enhancing the antibacterial effect.

A user may select the operating mode of the docking station 1 via an interactive module of the docking station or a mobile terminal. Alternatively, when the cleaning device 2 is docked in the docking station 1, the docking station 1 automatically activates the dust collection mode, and once the duration of the dust collection mode reaches a predefined time, the docking station 1 switches to the antibacterial mode.

Optionally, the dust collection mode and the antibacterial mode can be activated simultaneously. When both the dust collection mode and the antibacterial mode are activated, the fan 13 operates at the first power and the first rotational speed, and the antibacterial module 14 is activated, so that the antibacterial treatment is performed while the debris is rapidly collected from the dust collection box 21 of the cleaning device 2.

In an embodiment, as shown in FIG. 1, the docking station 1 further includes a cover 15, and the cover 15 covers an opening of the dust collection chamber 111, and is rotatably or removably connected to the housing 11 to open or close the dust collection chamber 111 to be opened or closed.

As shown in FIG. 1, the opening of the dust collection chamber 111 is located at the front side of the housing 11. When the cover 15 is provided at the opening of the dust collection chamber 111, it can seal the front side of the dust collection chamber 111.

By rotatably or removably connecting the cover 15 to the housing 11, the dust collection chamber 111 may be conveniently opened. Specifically, the dust collection chamber 111 contains a dust bag therein which is used to collect debris. After the dust collection chamber 111 is opened, the dust bag may be removed so that the debris may be taken out from the dust collection chamber 111, making it convenient to clean or replace the dust bag and preventing the debris from contaminating the interior of the docking station 1.

The present invention also provides a cleaning system which includes a cleaning device 2 and the docking station 1 provided in any of the above embodiments. The cleaning device 2 includes a dust collection box 21, and the dust collection box 21 is adapted to be connected to the dust collection duct 12 of the docking station 1.

The cleaning device 2 includes but is not limited to a sweeping robot, a mopping robot, a floor-washing machine, etc.

After the dust collection box 21 is connected to the dust collection duct 12 of the docking station 1, under the driving of the fan 13, air enters into the dust collection box 21 of the cleaning device 2 and carries away debris inside the dust collection box 21, and the debris is collected into the dust collection chamber 111 of the docking station 1 through the dust collection duct 12, thereby emptying the dust collection box 21 of the cleaning device 2. The air circulates and flows within the dust collection chamber 111, the dust collection duct 12 and the dust collection box 21, enabling the antibacterial module 14 to continuously perform an antibacterial treatment on the air within the dust collection chamber 111, the dust collection duct 12 and the dust collection box 21, which ensures a more comprehensive and thorough antibacterial effect

Furthermore, the dust collection duct 12 includes a dust suction duct 121 and a blowing duct 122, the dust suction duct 121 and the blowing duct 122 are respectively connected to the dust collection chamber 111, an air inlet end of the fan 13 is connected to the dust collection chamber 111, and an air outlet end of the fan 13 is connected to the blowing duct 122. The dust collection box 21 is provided with an air inlet 211 and an air outlet 212, the air inlet 211 is used to connect with the blowing duct 122, and the air outlet 212 is used to connect with the dust suction duct 121.

As shown in FIG. 3 and 4, when the docking station 1 is collecting dust, the fan 13 is activated, and a negative pressure is created inside the dust collection chamber 111 to output an air flow to the blowing duct 122. The air flow enters into the dust collection box 21 from the air inlet 211 of the cleaning device 2, and carries debris away from the dust collection box 21. The debris is collected into the dust collection chamber 111 of the docking station 1 through the air outlet 212 of the cleaning device 2 and the dust suction duct 121. The air flow circulates along and through the blowing duct 122, the dust collection box 21 of the cleaning device 2, the dust suction duct 121, and the dust collection chamber 111.

For example, when the antibacterial module 14 is at least partially located within the blowing duct 122, under the action of the antibacterial module 14, the air flow within the blowing duct 122 may carry antibacterial substances such as negative ions to flow through the blowing duct 122, the dust collection box 21 of the cleaning device 2, the dust suction duct 121, and the dust collection chamber 111, enabling the air flow carrying the antibacterial substances to be circulated, thereby achieving comprehensive and thorough antibacterial treatment.

For example, as shown in FIG. 4, the cleaning device 2 further includes a dust inlet cover 22 and a filter mesh 23 arranged opposite to each other. The dust inlet cover 22 and the filter mesh 23 are respectively provided on the front and rear sides of the cleaning device 2 to form a sealed air path, allowing the air to enter into the dust collection box 21 through the air inlet 211 of the dust collection box 21 and then flow out from the dust collection box 21 through the air outlet 212.

In the present disclosure, the terms "first'" and "second" are used solely for descriptive purposes and shall not be interpreted as indicating or implying relative importance. The term "a plurality of..." refers to two or more, unless otherwise explicitly specified.

## Claims

1. A docking station (1), comprising:
a housing (11) provided with a dust collection chamber (111);
a dust collection duct (12) connected to the dust collection chamber (111);
a fan (13) connected to both the dust collection chamber (111) and the dust collection duct (12), wherein the fan (13) is used to circulate air in the dust collection chamber (111) and the dust collection duct (12); and
an antibacterial module (14) at least partially located within the dust collection duct (12).

2. The docking station (1) according to claim 1, wherein the antibacterial module (14) comprises one or more of a negative ion generator, a disinfectant sprayer, a disinfectant sustained-release device, and an ultraviolet radiator.

3. The docking station (1) according to claim 1 or 2, wherein the dust collection duct (12) includes a dust suction duct (121) and a blowing duct (122), the dust suction duct (121) and the blowing duct (122) are respectively connected to the dust collection chamber (111), an air inlet end of the fan (13) is connected to the dust collection chamber (111), and an air outlet end of the fan (13) is connected to the blowing duct (122).

4. The docking station (1) according to claim 3, wherein the antibacterial module (14) comprises a main body (141) , an output line (142), and a release tip (143), the main body (141) is mounted on the housing (11), two ends of the output line (142) are respectively connected to the main body (141) and the release tip (143), and the release tip (143) extends into the dust collection duct (12).

5. The docking station (1) according to claim 4, wherein the main body (141) is located at a side of the blowing duct (122), and the release tip (143) extends into the blowing duct (122).

6. The docking station (1) according to claim 5, wherein the antibacterial module (14) further comprises a mounting bracket (144), the mounting bracket (144) is fixed to an outer wall of the blowing duct (122), and an end of the output line (142) close to the release tip (143) is inserted into the mounting bracket (144), so that the release tip (143) passes through the mounting bracket (144) and extends into the blowing duct (122).

7. The docking station (1) according to any one of claims 4 to 6, wherein the antibacterial module (14) is a negative ion generator, the output line (142) is a high-voltage output line, the main body (141) comprises a current conversion module connected to a power supply and configured to convert an input direct current or alternating current into negative high voltage direct current,
wherein the output line (142) is configured to transmit the negative high voltage direct current to the release tip (143) so that the release tip (143) generates an electric corona.

8. The docking station (1) according to claim 3, wherein the antibacterial module (14) has a business end configured to generate an antibacterial substance or perform antibacterial operation, wherein the business end is located within the dust suction duct (121) or the blowing duct (122).

9. The docking station (1) according to claim 8, wherein the docking station (1) comprises a plurality of the antibacterial modules (14), the business ends of a part of the antibacterial modules (14) are extended into the blowing duct (122), and the business ends of the other part of the antibacterial modules (14) are extended into the dust suction duct (121).

10. The docking station (1) according to any one of claims 1 to 9, wherein the docking station (1) has a dust collection mode and an antibacterial mode, the fan (13) is configured to switch between the dust collection mode and the antibacterial mode, the antibacterial module (14) is configured to be activated at least in the antibacterial mode, and a power of the fan (13) in the dust collection mode is greater than a power of the fan (13) in the antibacterial mode.

11. The docking station (1) according to any one of claims 1 to 10, wherein the docking station (1) further comprises a cover (15), and the cover (15) covers an opening of the dust collection chamber (111), and is rotatably or removably connected to the housing (11) to open or close the dust collection chamber (111).

12. A cleaning system, comprising a cleaning device (2) and the docking station (1) according to any one of claims 1 to 11, wherein the cleaning device (2) comprises a dust collection box (21), and the dust collection box (21) is adapted to be connected to the dust collection duct (12) of the docking station (1).

13. The cleaning system according to claim 12, wherein the dust collection duct (12) comprises a dust suction duct (121) and a blowing duct (122), the dust suction duct (121) and the blowing duct (122) are respectively connected to the dust collection chamber (111), an air inlet end of the fan (13) is connected to the dust collection chamber (111), and an air outlet end of the fan (13) is connected to the blowing duct (122); and
the dust collection box (21) is provided with an air inlet (211) and an air outlet (212), the air inlet (211) is used to connect with the blowing duct (122), and the air outlet (212) is used to connect with the dust suction duct (121).

14. The cleaning system according to claim 12 or 13, wherein the housing (11) further comprises an accommodation section (112) at the bottom of the dust collection chamber (111), and the accommodation section (112) has a shape matching a shape of the cleaning device (2), so that the cleaning device (2) can rest against the accommodation section (112).
